# EUROPEAN PATENT APPLICATION

(11) **EP 1 882 938 A2**
(43) Date of publication of application: **30.01.2008**
(21) Application number: 07014644.4
(22) Date of filing: 26.07.2007
(51) Int. Cl.: G01N 33/542

(54) **Method and apparatus for the fast determination of deoxynivalenol in a cereal-based matrix**

(30) Priority: 28.07.2006 IT MI20061502
(71) Applicant: Barilla G. e R. Fratelli S.p.A., 43100 Parma (IT); Consiglio Nazionale delle Ricerche, 00185 Roma (IT)
(72) Inventor: Visconti, Angelo c/o Consiglio Nazionale delle Ricerche, 70126 Bari (IT); Pascale, Michelangelo c/o Consiglio Nazionale delle Ricerche, 70126 Bari (IT); Lippolis, Vincenzo, 70017 Putignano (Bari) (IT); Ranieri, Roberto, 43100 Parma (IT); Silvestri, Marco, 43100 Parma (IT); D'Alessandro, Alessandro, 43100 Parma (IT)
(74) Representative: Ferreccio, Rinaldo

(57) **Abstract**

The invention relates to a method for DON determination in a cereal-based matrix based on the Fluorescence Polarization Immunoassay (FPIA) technique, wherein the fluorescence polarization measurement performed on the matrix is compared with a polarization value corresponding to a known DON concentration and then corrected by a predetermined quantity (overestimation) depending on the nature and type of cereal-based matrix.

## Description

### Field of application

In its more general aspect, the present invention relates to the food safety field and particularly the invention relates to a method and apparatus for the determination of mycotoxins in a cereal-based matrix.

More particularly, the invention relates to a method as above based on the Fluorescence Polarization Immunoassay (FPIA) technique for determination of deoxynivalenol (DON) in such matrices.

The term cereal-based matrix is used herein to indicate a crude, semifinished or finished food product based on cereals, mentioning among them, by way of example, wheat, maize, barley, oats, rye and unprocessed caryopses in general, bran, semolina and flours, breakfast cereals, pasta, biscuits, bread and similar bakery products based on cereals

### Prior art

It is known that mycetes and moulds can develop in some kinds of foodstuffs, being able to generate in turn mycotoxins that can produce toxic effects on human beings and animals.

In particular, fungi can develop and mycotoxins can be formed both in the field on the plant and in any one of the subsequent foodstuff storage and transformation steps

Mycotoxin toxicity, occurring under different forms, includes acute, cancerogenic, teratogenic, estrogenic, immunosuppressant and neurologic toxic effects, appearing according to the kind of mycotoxin, dose, organ being concerned, age, sex and species of the organism taking the micotoxin.

Due to mycotoxin toxicity, health autorities enforced the systematic control thereof for some of them, through EEC regulations, to control that they do not exceed predetermined threshold levels in food and feedstuff.

Also other international organizations, such as the United Nations Food and Agricolture Organization (FAO), the Food and Drug Administration (FDA) and the World Health Organization (WHO) engaged on different fronts among which the assessment of the risk for human and animal health deriving from the exposure to mycotoxins.

The most common mycotoxins include trichothecenes and particularly deoxynivalenol (DON), also called vomitoxin, which i.a. causes vomiting, lack of appetite and immunosuppression. DON is produced by F. graminearum and F. culmorum, fungi of the Fusarium type.

European law, by (CE) Regulation no. 856/2005 of the Commission dated June 6, 2005, set the highest admissible DON contamination values in cereals and derivatives thereof, as from July 1, 2006, comprised in the range between 0.20 µg/g (in baby foodstuffs) and 1.75 µg/g (for unprocessed hard wheat).

From what illustrated above it derives the need to have fast and reliable analysis methods, in terms of accuracy and precision, to control DON content in food and feedstuff.

Prior art, to meet this requirement, provides several chromatographic methods, among which the one based on high pressure liquid chromatography is among the most used and reliable.

Without describing this method in detail, being the chromatographic process well known, it must be said that, although advantageous under certain aspects, it has some drawbacks among which long execution times (some hours for a complete analysis) making it unsuitable for use in a production and/or transformation cycle of a food product based on cereals.

Moreover, this method is caracterized by high costs due to the complex analyses to be performed and to the instruments used and also to the use of skilled workers, who have to be suitably trained.

Moreover, it is implemented through bulky apparatus, which are hard to transport, also because they are very fragile.

Patent application WO 02/23196, incorporated herein by reference, discloses a method for DON detection, which is based on the Fluorescence Polarization Immunoassay technique and allows molecular interactions to be studied, by monitoring the molecular size changes of a fluorescent molecule.

In particular, the Fluorescence Polarization Immunoassay test (FPIA) is used, wherein the molecular size changes of a fluorescent tracer result from an antigen-antibody interaction, wherein the antigen corresponds to the toxin to be quantified or to a tracer consisting of the toxin bound to a particular fluorescent "probe".

For a small fluorescent molecule, such as the tracer being used, a low polarization is obtained, being caused by a fast rotation, viceversa, big-sized fluorescent molecules, such as the ones formed by a tracer/antibody complex, determine a high polarization because of their lower rotation speed.

The presence of free DON in solution causes a competition reducing the quantity of antibody that binds to the tracer and consequently reducing the polarization value.

The polarization value is thus inversely proportional to DON content and allows the quantitation thereof.

In detail, WO 02/23196 describes as the antibodies being used two DON-specific monoclonal antibodies identified by the clone reference number #1 and #4, and a DON fluorescent derivative obtained by binding DON to 6-amino-fluorescein by means of 1,1-carbonyl diimidazole.

Although this method is advantageous for some reasons, it is not a valid alternative to the more consolidated HPLC method since the results obtained by comparing the two methods did not show a good correlation, as confirmed by tests performed in the Applicant's laboratories.

Moreover this FPIA method proved to suffer from an unsatisfactory sensitivity, about 0.50 µg/g as deducible from patent WO 02/23196, which makes it suitable mostly for use as screening method for DON detection in unprocessed wheat samples, but not correctly applicable to the analysis of products derived therefrom (for example semolina, pasta and baby food) whose highest admissible levels are lower or close to the detectability limit of the method itself.

### Summary of the invention

A first aim of the present invention is thus to provide an alternative method to the ones provided by prior art to determine (detection and quantitation) deoxinyvalenol (DON), which allows in particular this determination in a cereal-based matrix.

A second aim of the present invention is to provide a method of the above type, which is particularly fast allowing DON presence to be detected in real time during a production and/or transformation process of a cereal-based food product.

A third aim of the present invention is to provide a method of the above type, which can be completely integrated in a food chain or a production and/or transformation cycle of a cereal-based food product.

A further aim of the present invention is to provide a process for DON determination being particularly accurate, precise and sensitive.

A further aim of the present invention is to provide a process of the above type which is particularly simple and economically advantageous.

These aims are reached, according to the invention, by a method based on the Fluorescence Polarization Immunoassay (FPIA) technique for DON determination in a cereal-based matrix, comprising the steps of:
a) providing a solution of a filtered extract obtained from a predetermined cereal-based matrix;
b) providing an antibody and a tracer comprising DON conjugated with a predetermined fluorophore;
c) mixing predetermined quantities of said filtered extract solution and of said antibody to obtain a solution corresponding to the reading blank of a fluorescence polarization measurement;
d) performing after a predetermined incubation time a fluorescence polarization measurement of said solution corresponding to the reading blank;
e) adding to said solution corresponding to the reading blank a predetermined quantity of said tracer, which is able to bind to said antibody to determine a detectable fluorescence polarization change;
f) performing after a predetermined incubation time a fluorescence polarization measurement of said solution;
g) comparing said measured fluorescence polarization with a typical fluorescence polarization value, said typical fluorescence polarization value corresponding to a known DON concentration, thus obtaining a first DON concentration value;
h) changing by a predetermined quantity the obtained first DON concentration value, said predetermined quantity being correlated to the nature and type of said cereal-based matrix.

According to the invention, said obtained first DON concentration value is changed by a quantity comprised between 0,60 and 0,02 micrograms of DON per grams of cereal-based matrix.

Therefore, for example, if the cereal-based matrix is composed of unprocessed hard wheat caryopses, said predetermined quantity to be subtracted from said first DON concentration value being detected is comprised between 0,35 and 0,04 µg/g, and even more particularly, for milled hard wheat whose particle size is lower or equal to 1 mm, it is equal to 0.27±0.03 µg/g.

It must be noted that said value is independent from the variety of wheat being examined but, according to the invention, said predetermined quantity changes when the matrix type and nature changes.

In the unprocessed soft wheat caryopses case, said quantity to be subtracted from the first DON concentration value being obtained is comprised between 0.50 and 0.04 µg/g, and more particularly for milled wheat whose particle size is lower or equal to 1 mm, it is equal to 0.39±0.06 µg/g.

In the semolina case, the quantity to be subtracted to the first DON concentration value being obtained is comprised between 0.10 and 0.04 µg/g and more particularly it is equal to 0.08±0.01 µg/g, while if the cereal-based matrix is composed of pasta the overestimation is comprised between 0.06 and 0.02 µg/g and more particularly it corresponds to 0.04±0.01 µg/g.

In the particular case of a cereal-based matrix composed of bran, and in other cases such as for example for maize, said quantity to be subtracted from the first DON concentration value being obtained is particularly high, and out of range, with respect to the previously indicated values.

This apparent variance is determined by the presence of interfering sustances being co-extracted with DON which, nevertheless, according to the invention, can be removed from the filtered extract through a further purification stage.

In these cases wherein DON overestimation is particularly high, and generally comprised between 1.50 and more than 2.00 micrograms of DON per grams of matrix, but nevertheless according to the invention even when DON overestimation is comprised in said range of values between 0.60 and 0.02 micrograms of DON per grams of cereal-based matrix, the overestimation can be minimized, or even completely removed, by means of a purification with activated charcoal or other adsorbent material, as it will be apparent from the following description.

Preferably, said fluorophore is 4'-amino-methyl-fluorescein reacting with DON to form said tracer indicated with DON-FL2, which reacts with the monoclonal antibody having the clone reference number #22, advantageously used in the method according to the invention and being hereafter simply indicated with antibody #22.

In particular, antibody #22, which proved to be particularly sensitive, has been directly provided by Dr. C. Maragos (USDA, USA) and purified as described in Maragos, C. M., and McCormick, S. P., 2000, Monoclonal antibodies for the mycotoxins deoxynivalenol and 3-acetyl-deoxynivalenol. Food and Agricultural Immunology, 12, 181-192.

Advantageously, DON-FL2 is selectively obtained as the sole DON fluorescent derivative by protecting DON hydroxylic groups at C7 and C 15 through a reaction with 1-butyl-boronic acid, before its conjugarion with the fluorophore.

In the case of a solid matrix, such as for example unprocessed caryopses, semolina and pasta as above, but also in the case of flour, breakfast cereals, bread, biscuits and the like, said extract is obtained by extraction of said matrix in a PBS buffer and following filtration being repeated at least once.

In particular, the matrix is at first finely divided, then energetically agitated in such PBS solution for about 2-5 minutes, then extracted.

In a preferred embodiment of the present method two filtrations are provided, a first filtration on a paper filter and a second filtration on a glass microfiber filter, with no need for further purifications of the so-obtained filtered extract.

In the particular case of the solid matrix made of bran, as above described, said purification stage is expected to occur after the filtrations and before fluorescence polarization measurement.

In practice, in this stage, the use of activated charcoal or other adsorbent materials allows to pull down the signal due to co-extracted interfering substances, and, depending on the quantity of adsorbent material being used, the overestimation is minimized or removed.

Therefore, for example, for concentrations up to about 1.0-1.5 mg of activated charcoal for mL of filtered extract, a minimization of the detected overestimation is obtained.

In the case in which the filtered extract is purified by addition of activated charcoal in a quantity comprised between 2 and 3 milligrams per milliliter of extract, the overestimation is instead substantially removed.

In any case, the addition of adsorbent material is followed by agitation and subsequent filtration, proceeding the fluorescence polarization value determination, as previously mentioned with reference to steps a-g of the present method.

It must be noted that, in another aspect thereof, the present invention relates to an apparatus for executing the above described method, herein also indicated with automatic sampler, capable of automating the present method through a software management.

In particular this apparatus comprises:
at least a syringe comprising a side union, an active wash flow pump and a needle with a predetermined length;
a powered unit for moving said syringe by means of a rotary shaft and a mechanical arm;
at least a drain well to wash said needle;
a first plurality of housings for respective test tubes and/or vials intended to respectively contain the buffer, the antibody and the tracer in solution to be drawn in predetermined quantities by means of the syringe;
a second plurality of housings for respective test tubes and/or vials intended to contain each one a solution of a filtered extract being obtained from one or more cereal-based matrices to be analysed, to be drawn in a predetermined quantity by means of said syringe;
a fluorescence polarization reader provided with a reading cell equipped with a covering lid and a reading cuvette suitable to be housed in said reading cell;
a motor suitable to move said covering lid;
a computer-controlled management software.

According to a preferred embodiment, the apparatus according to the invention also comprises an automatic cuvette exchange system, to remove the reading cuvette after measuring the quantity of DON contained in a first sample, i.e. a solution of a first filtered extract, and to replace it with a new cuvette to perform a further reading on a different sample such as a solution containing a second filtered extract.

In particular, the cuvette exchange system comprises grasping means, preferably pliers-like, positioned on said syringe near the needle, or however associated to the mechanical arm at a needle-guiding portion thereof.

Even more particularly, these grasping means are suitable to directly grasp a cuvette or, preferably, they are suitable to grasp a holed stopper which can be associated to the cuvette and which enables to grasp and transport it.

In a further preferred embodiment of the apparatus according to the invention, the present automatic sampler also comprises an automatic filtration system, to obtain said filtered extract from a solution containing the finely divided cereal-based matrix to be tested.

It must be said that, advantageously, the present method has provided results, which are in perfect accordance with the ones obtained from a comparative HPLC analysis, particularly for wheat-based matrices.

Moreover, the method according to the invention has proved to be particularly rapid and more accurate than HPLC method, with a precision that is comparable and even better than that of the comparative HPLC method.

Further advantages and features of this invention will be apparent from the description of some embodiments of a method according to the invention, made hereafter with reference to the annexed drawings, given by way of non limiting examples.

### Brief description of the drawings

In the drawings:

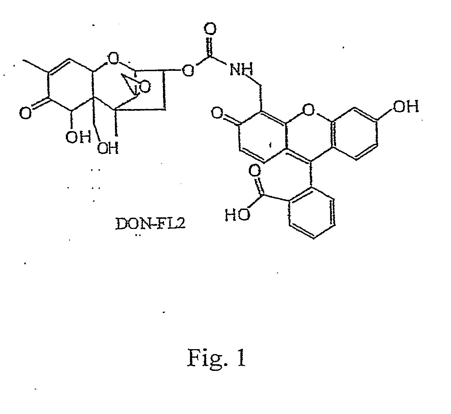

- Figure 1 shows the structural formula of a DON fluorescent derivative used as a tracer in the method according to the invention;

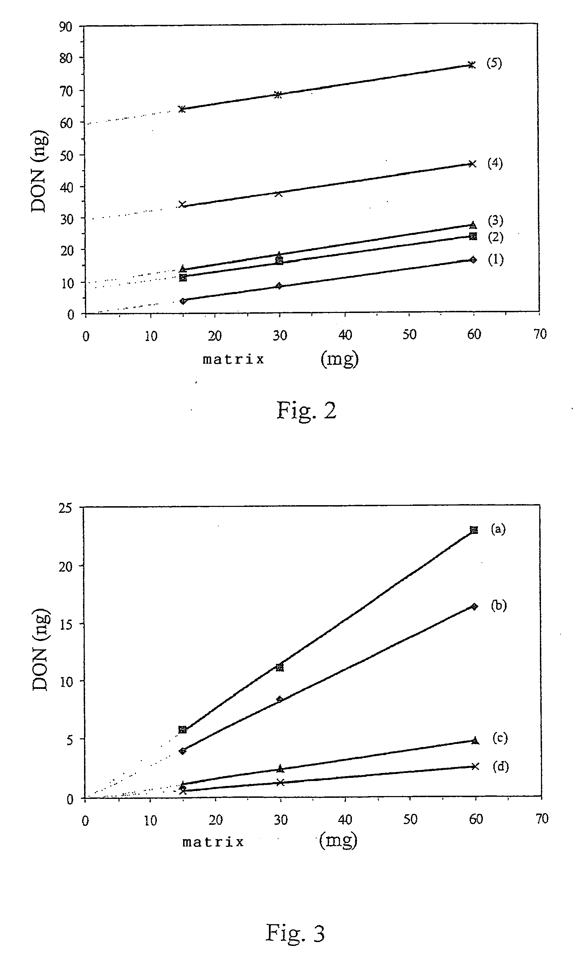

- Figure 2 shows the matrix effect on DON content being determined through FPIA wherein the broken line 1) is obtained for an extract from unprocessed and DON-uncontaminated hard wheat caryopses, while broken lines 2)-5) are obtained for extracts from caryopses of hard wheat that are DON-contaminated at levels of 7.5, 10, 30, 60 ng respectively.
- figure 3 shows the matrix effect on DON content being determined through FPIA wherein broken lines show the linear regression obtained respectively for a) soft wheat caryopses; b) hard wheat caryopses; c) semolina; d) DON-uncontaminated pasta.
- figure 4 graphically shows the correlation between DON content in naturally-contaminated matrix samples, analysed through the method according to the invention and through the comparative HPLC method, respectively for: A) 35 samples of unprocessed hard wheat caryopses; B) 32 samples of unprocessed soft wheat caryopses; C) 22 semolina samples; D) 26 pasta samples.

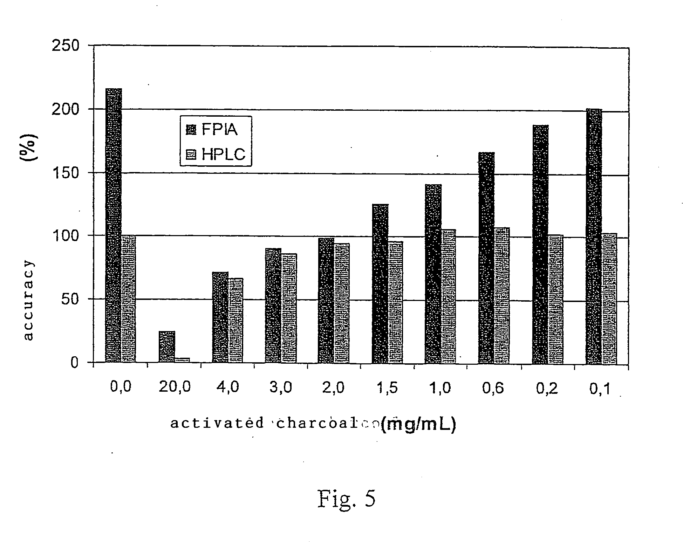

- figure 5 graphically shows the accuracy (expressed in terms of DON percentage concentration) obtained by using different activated charcoal concentrations in DON determination in the same bran sample through the FPIA and HPLC methods.
- figure 6 schematically shows an apparatus for the automated implementation of the method according to the invention.

### Detailed description

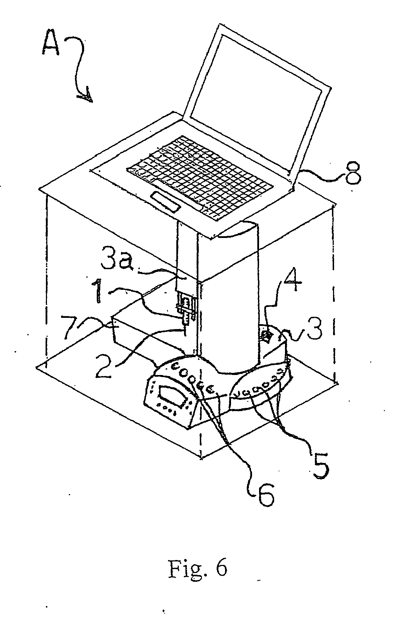

With reference to figure 6, an apparatus for the automated implementation of the method according to the present invention is globally indicated with A and also defined as automatic sampler.

The automatic sampler A comprises at least a syringe 1 with a capacity preferably ≥ 1mL, equipped with a side union, an active washflow pump and a preferably ≥ 80 mm-long needle 2.

The automatic sampler A also comprises a powered unit 3, suitable to move said syringe 1 by means of a rotary shaft and a mechanical arm schematized in the figure and globally indicated with 3a.

For the automatic cleaning of needle 2, the automatic sampler A is equipped with at least a drain well 4 wherein the needle is washed both internally and externally.

The automatic sampler A also comprises a first plurality of housings 5 for respective test tubes and/or vials intended to contain a buffer solution, an antibody solution and a tracer solution, wherefrom respective predetermined quantities are drawn by means of the syringe 1, as it will be apparent from the following description.

A second plurality of housings 6, provided in the automatic sampler A according to the invention, is instead intended to house test tubes and/or vials comprising each one a sample to be analysed, i.e. a solution of a filtered extract obtained from one or more cereal-based matrices to be analysed.

In order to determine the quantity of DON contained in the sample(s), the automatic sampler A is equipped with a fluorescence polarization reader 7, such as for example a fluorometer or a spectrofluorometer provided with polarizing filters, provided with a reading cell equipped with a covering lid and with at least a reading cuvette suitable for housing in the reading cell.

The covering lid is moved by a respective motor, moving this lid from an open position, allowing the reading cuvette to be housed in and removed from the respective cell, to a reading cell closed position.

According to the invention, the automatic sampler A is programmable through a computer 8, or in a serial way, and it is managed to implement the method according to the invention through a software.

According to a preferred embodiment, the automatic sampler A also comprises an automatic cuvette exchange system, to remove the reading cuvette after measuring the quantity of DON contained in the sample solution, and to replace it with a new reading cuvette to perform a subsequent analysis on a further filtered extract.

In particular, the cuvette exchange system comprises grasping means, for example pliers-like, positioned on the syringe 1 near the needle 2, or however associated to the mechanical arm at a needle-guiding portion thereof.

Even more particularly, grasping means are suitable to directly grasp a cuvette or, preferably, they are suitable to grasp a holed stopper which can be associated to the cuvette and which enables to grasp and transport it.

In this case, in the automatic sampler A a plurality of reading cuvettes are housed in said housing seats for test tubes and/or vials, for example in the second plurality of housing seats 6 for the test tubes containing the samples.

In a further preferred embodiment of the apparatus according to the invention, the present automatic sampler also comprises an automatic filtration system, to obtain said filtered extract from a solution containing the finely divided cereal-based matrix to be tested.

According to the invention, said automatic sampler A allows the present method for DON determination in a cereal-based matrix to be automated, as described hereafter.

In practice, once the solutions comprising the sample(s), the buffer, the antibody and the tracer have been provided and the test tubes comprising these solutions positioned in the respective housings, and once a reading cuvette has been positioned in the proper cell of the fluorescence polarization reader, the automatic sampler will automatically perform the following phases, after a start command:
- drawing through the syringe 1 a predetermined quantity of buffer solution and injecting it into the reading cuvette;
- drawing through the syringe 1 a predetermined quantity of antibody solution and injecting it into the reading cuvette;
- drawing through the syringe 1 a predetermined quantity of solution containing the filtered extract and injecting it into the reading cuvette;
- washing the syringe with the buffer solution;
- incubating for about 2 minutes under agitation the obtained solution, which corresponds to the reading blank;
- closing the reading cell by means of the respective lid and measuring the fluorescence polarization in said solution, which corresponds to the reading blank;
- opening the reading cell covering lid;
- drawing through the syringe 1 a predetermined quantity of solution containing the tracer and injecting it into the reading cuvette containing the solution corresponding to the blank;
- washing the syringe with the buffer solution;
- incubating for about 2 minutes under agitation the obtained solution contained in the reading cuvette;
- closing the reading cell by means of the respective lid and measuring the fluorescence polarization in the thus obtained solution;
- acquiring, processing and filing the obtained data.

Preferably, the fluorescence polarization measurement in the obtained solution is repeated in triplicate in order to improve the accuracy of the data, which is consequently processed as the average value of three measurements performed on the same solution. In practice, once a first DON measurement is achieved, the automatic sampler will be able to start subsequent measurements both following a new start command and when it has been programmed to perform serial measurements, optionally by automatically replacing the reading cuvette as previously described.

Without wishing to limit the scope of the present invention, the following examples show how to implement and use the present invention.

In particular, in the examples mentioned hereafter reference is made to:
- Example 1: four wheat-based matrices corresponding to unprocessed hard wheat caryopses, unprocessed soft wheat caryopses, semolina and pasta, but it must be understood that other matrices, such as for example, but not only, bread, even from different cereals, can be used in the method according to the present invention to determine the DON content thereof;
- Example 2: bran, but it must be understood that other matrices, such as maize for example, can be used in the method according to the present invention to determine the DON content thereof.

### EXAMPLE 1 (foreseeing the direct overestimation subtraction)

First of all, in order to obtain the filtered extract of a wheat-based matrix, a quantity of 25 g of matrix (of caryopses, semolina and pasta) is finely milled through a Buehler MLI 204 mill by Buehler S.p.A., Milan; Italy, sifted with a 1 mm-mesh screen and subjected to an energetic agitation step (blending) in 100 mL of PBS buffer for three minutes.

An extract is thus obtained, sequentially undergoing a first filtration on a Whatman N. 4 paper filter, and a second filtration on a Whatman GF/A glass microfiber filter.

The obtained filtered extract undergoes then the analysis for DON measurement through the Fluorescence Polarization Immunoassay (FPIA) technique by using a PerkinElmer LS 55 spectrofluorometer.

The fluorescence polarization measurement is performed by using the tracer DON-FL2 obtained by DON reaction with 4'-amino-methyl-fluorescein and the monoclonal antibody having the clone reference number #22.

The tracer DON-FL2 was obtained by bringing some advantageous changes to the method described by Dr. C. Maragos in "Maragos, C. M.; Plattner, R. D. Rapid fluorescence polarization immunoassay for the mycotoxin deoxynivalenol in wheat. J. Agric. and Food Chem. 2002, 50, 1827-1832".

In detail, the steps of protecting DON hydroxy groups in C1 and C15 through a reaction with butyl-boronic acid to form a cyclic boron ester have been added to said method.

Then the activation of the hydroxy group in C-3 with 1,1-carbonyl diimidazole and the following deprotection in C7 and C15 are carried out.

The activated DON is then reacted with 4'-amino-methyl-fluorescein hydrochloride to form DON-FL2.

The diagram reported hereafter shows the reactions through which DON-FL2 is obtained.

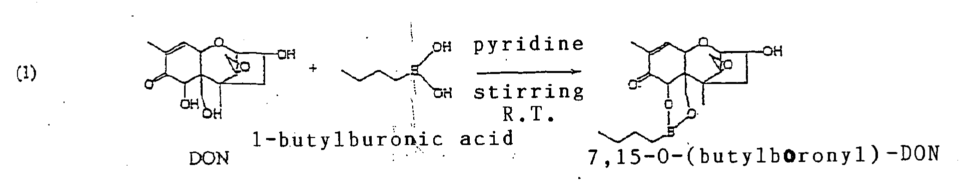

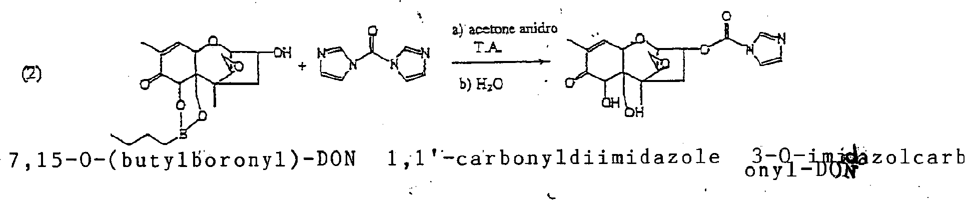

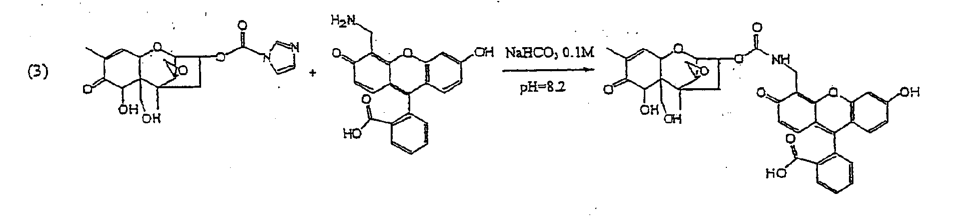

In order to evaluate how the kind of matrix affects the results of DON quantity being detected by means of FPIA method, actions have been then taken as follows.

Filtered extracts from ten samples of hard wheat caryopses matrices of different varieties have been obtained in the above manner, proving to be DON-uncontaminated through HPLC analysis, and contaminated with known DON quantities at different contamination values.

The FPIA measurement on the solutions always provided an overestimation of DON content in solution (matrix effect) which proved to be almost constant at the different contamination levels and when varying the wheat variety being considered.

The same study was carried out on semolina and pasta matrix samples and the different matrix effects have been quantified (Table 1).

**Table 1. Average overestimations obtained on 10 different varieties of samples of unprocessed carioxide, semolina and pasta matrices.**

| MATRIX | **Overestimation (µg/g)** |
|---|---|
| Unprocessed hard wheat caryopses | 0.27 ± 0.03 |
| Unprocessed soft wheat caryopses | 0.39 ± 0.06 |
| Semolina | 0.08 ± 0.01 |
| Pasta | 0.04 ± 0.01 |

Since in the same matrix (caryopsis, semolina and pasta) this matrix effect proved to be constant, it is possible to correct the DON concentration values detected in a FPIA analysis on a sample of a predetermined matrix, and expressed in µg/g, by subtracting the obtained overestimation.

Considering the matrix quantity used for measuring, in the described particular case amounting to 60 mg, the FPIA analysis linearity range can be defined between 0.10 and 2.0 µg/g, expressed as DON quantity per gram of sample.

- Concerning the comparison between the result of the measured fluorescence polarization of a sample solution obtained through FPIA and a characterized fluorescence polarization value corresponding to a known DON concentration, it must be said that the known concentration is determined through HPLC method.

In particular, in order to evaluate the FPIA method recovery values, sample matrices (of caryopses, semolina and pasta), being artificially contaminated with known DON quantities at three different levels (0.25; 0.75; 1.25; 1.75 µg/g), have been analyzed in triplicate.

Respective filtered extracts have been obtained from these matrices as above described, after said contamination, and each filtered extract being obtained has undergone in parallel both a FPIA analysis and a HPLC analysis.

The results obtained from FPIA analysis (Table 2) have shown, for the different analyzed matrices, a method average recovery close to 100% (98-102%) and a low variability while the average recovery obtained from HPLC analysis proved to be lower (82-87%) and with a higher variability.

**Table 2. DON recovery values from unprocessed hard wheat and soft wheat caryopses, semolina and pasta both for FPIA and HPLC method.**

| Spiking level(µg/g) | recovery ±SD^{a} (%) | | | | | | | |
|---|---|---|---|---|---|---|---|---|
| | hard wheat | | semolina | | Pasta | | soft wheat | |
| | HPLC | FPIA | HPLC | FPIA | HPLC | FPIA | HPLC | FPIA |
| 0.25 | 82.5±9.9 | 97.2±3.6 | 83.5±4.4 | 100.7±3.5 | 87.2±1.9 | 99.8±5.3 | 95.4±2.1 | 95.6±7.8 |
| 0.75 | 79.9±6.8 | 96.1±5.5 | 87.4±3.5 | 106.6±4.8 | 87.5±2.0 | 101.5±1.6 | 92.6±2.4 | 108.2±3.2 |
| 1.25 | 84.2±8.0 | 100.1±2.9 | 87.6±2.8 | 98.8±1.7 | 87.6±1.3 | 102.3±2.8 | 89.8±1.4 | 99.7±1.8 |
| 1.75 | 83.2±8.1 | 97.5±4.1 | 85.6±3.1 | 102.1±3.0 | 86.3±3.0 | 99.5±4.1 | 88.7±2.3 | 100.5±2.1 |
| average ±SD | 82.2±7.5 | 97.8±4.0 | 86.2±3.7 | 102.0±4.7 | 87.4±1.5 | 101.2±3.3 | 91.6±3.0 | 101.0±5.3 |

- On the contrary, in order to evaluate the correlation between the two methods being compared, 35 unprocessed hard wheat caryopses matrices, 32 unprocessed soft wheat caryopses matrices, 22 semolina matrices and 26 pasta matrices being contaminated by nature have been analysed.

Matrices have been extracted with PBS and the filtered extract has undergone in parallel both a FPIA analysis and a HPLC analysis.

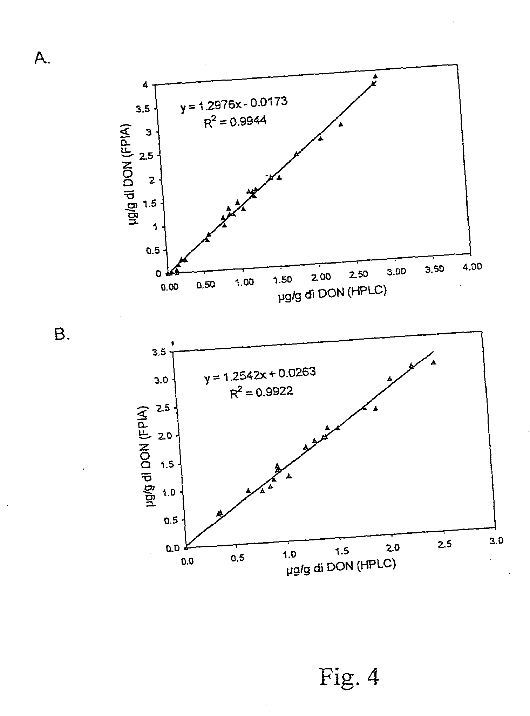

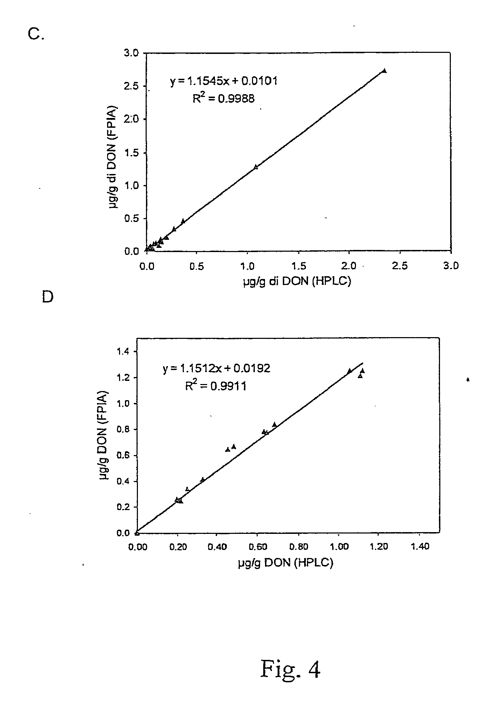

For all matrices wherein DON concentration was higher than 2,00 µg/g it was necessary to repeat the FPIA measurement after a suitable extract diluition to bring the concentration value back to the method linearity range. A good correlation occurred between FPIA and HPLC analyses, the two methods being compared in the analysis of the naturally-contaminated matrix samples (figure 4), except for the line slope, which does not prove to be unitary and which would involve an overestimation of DON content mainly at high contamination levels.

But this apparent divergence is justified by the different average percentage recovery shown by the two different analysis techniques being compared.

In fact, by correcting the results by the respective recoveryvalues, an angular coefficient of the correlation lines is obtained, which is almost unitary and respectively equal to 1.0381 for hard wheat caryopses, 1.1538 for soft wheat caryopses, 0.9900 for semolina, and 1.0042 for pasta, expressing the perfect correspondence between the results obtained with the two analytic techniques.

### EXAMPLE 2 (foreseeing the use of activated charcoal to minimize or remove overestimation)

First of all, in order to obtain the bran matrix filtered extract, a quantity equal to 25 g is finely milled through a Buehler MLU 202 mill by Buehler S.p.A., Milan; Italy, and then subjected to an energetic agitation step (blending) in 200 mL of PBS buffer for three minutes.

An extract is thus obtained, sequentially undergoing a first filtration on a Whatman N. 4 paper filter, and a second filtration on a Whatman GF/A glass microfiber filter.

The filtered extract thus obtained is charged with a predetermined quantity of activated charcoal, subjected to agitation and subsequent filtration. The purified solution undergoes the polarization determination procedure as above reported.

- In order to evaluate how to remove or minimize the signal due to co-extracted interfering substances involving a considerable overestimation of bran DON content, actions have been then taken as follows.

Filtered extracts from the same naturally DON-contaminated bran sample have been obtained in the above manner and suitably treated with variable quantities of activated charcoal. Activated charcoal concentration in the filtered extract varies in the range 0-20 mg/mL.

The obtained purified extracts have undergone DON content determination through-FPIA and HPLC analyses.

The obtained results (Table 3) have shown that bran DON concentration, determined by FPIA method, is overestimated in the range 0-1.5 mg of activated charcoal per mL of filtered extract, with respect to the values obtained through HPLC analysis, which result to be not significantly different from each other. In the activated charcoal concentration range 2-3 mg/mL a good correspondence occurred between DON values obtained through FPIA and HPLC analyses and the one determined through HPLC analysis without activated charcoal. With activated charcoal concentrations exceeding 3 mg/mL a sharp decrease was shown for the DON value determined through FPIA and HPLC analyses and for the one determined through HPLC analysis without activated charcoal.

**Table 3. Activated charcoal concentration effect on DON values (µg/g) determined through FPIA and HPLC analyses in the same bran sample.**

| Activated charcoal (mg/mL) | DON concentration (µg/g) | |
|---|---|---|
| | FPIA | HPLC |
| 0 | 3.48 | 1.61 |
| 0.1 | 3.25 | 1.67 |
| 0.2 | 3.04 | 1.65 |
| 0.6 | 2.69 | 1.74 |
| 1.0 | 2.28 | 1.71 |
| 1.5 | 2.03 | 1.56 |
| 2.0 | 1.59 | 1.47 |
| 3.0 | 1.46 | 1.35 |
| 4.0 | 1.15 | 1.07 |
| 20.0 | 0.40 | 0.11 |

Therefore, advantageously, in the bran-based matrix case, the overestimation in DON determination can be directly removed through said purification stage by charging said filtered extract with a quantity of activated charcoal being comprised between 2 and 3 milligrams per millilitre of extract, with subsequent agitation and filtration.

### Advantages

A first advantage of the FPIA method for DON determination according to the invention is thus the highest accuracy shown with respect to the traditional HPLC method taken as a reference.

A second advantage is its sensitivity, equal to 0.1 µg/g, at least 5 times higher than the sensitivity being deducible from patent WO 02/23196.

It must also be said that the present method accuracy is comparable , if not better than that of said HPLC method.

A further main advantage of the method according to the invention is its execution speed, about 10-15 minutes, with reduced implementing costs.

The implementation of the present method by means of the automatic sampler according to the invention is then particularly advantageous, because it allows the method for the rapid determination of DON in a cereal-based matrix, particularly wheat, to be integrated in any food chain or production and/or transformation cycle of a crude, semifinished or finished cereal-based food product, thus ensuring a rapid and constant monitoring of the food product and an improved food safety.

Due to all the above advantages, the method according to the present invention is a valid alternative to the methods provided by the prior art for DON determination, and particularly an alternative to the HPLC method for DON determination in wheat-based food products.

## Claims

1. A method for DON determination in a cereal-based matrix, comprising the steps of:
a) providing a solution of a filtered extract obtained from a predetermined cereal-based matrix;
b) providing an antibody and a tracer comprising DON conjugated with a predetermined fluorophore;
c) mixing predetermined quantities of said filtered extract solution and of said antibody to obtain a solution corresponding to the reading blank of a fluorescence polarization measurement;
d) performing after a predetermined incubation time a fluorescence polarization measurement of said solution corresponding to the reading blank;
e) adding to said solution corresponding to the reading blank a predetermined quantity of said tracer, which is able to bind to said antibody to determine a detectable fluorescence polarization change;
f) performing after a predetermined incubation time a fluorescence polarization measurement of said solution;
g) comparing said measured fluorescence polarization with a charachterized fluorescence polarization value, said **characterized** fluorescence polarization value corresponding to a known DON concentration, thus obtaining a first DON concentration value;
h) changing by a predetermined quantity the obtained first DON concentration value, said predetermined quantity being correlated with the nature and type of said cereal-based matrix.

2. A method according to claim 1, wherein the obtained first DON concentration is changed by a quantity comprised between 0.60 and 0.02 micrograms of DON per grams of cereal-based matrix.

3. A method according to claim 1 or 2, wherein said cereal-based matrix consists of hard wheat caryopses and said predetermined quantity is comprised between 0.35 and 0.04 µg/g.

4. A method according to claim 3, wherein said predetermined quantity is equal to 0.27±0.03 µg/g.

5. A method according to claim 1 or 2, wherein said cereal-based matrix consists of soft wheat caryopses and said predetermined quantity is comprised between 0.50 and 0.04 µg/g.

6. A method according to claim 5, wherein said predetermined quantity is equal to 0.39±0.06 µg/g.

7. A method according to claim 1 or 2, wherein said cereal-based matrix consists of wheat semolina and said predetermined quantity is comprised between 0.10 and 0.04 µg/g.

8. A method according to claim 7, wherein said predetermined quantity is equal to 0.08±0.01 µg/g.

9. A method according to claim 1 or 2, wherein said cereal-based matrix consists of pasta and said predetermined quantity is comprised between 0.06 and 0.02 µg/g.

10. A method according to claim 9, wherein said predetermined quantity is equal to 0.04±0.01 µg/g.

11. A method according to claim 1, which further includes a purification stage, through the addition of an adsorbent material to said filtered extract before the latter is mixed with said antibody.

12. A method according to claim 11, wherein said cereal-based matrix consists of bran or maize.

13. A method according to claim 12, wherein said adsorbent material is activated charcoal.

14. A method according to claim 13, wherein said activated charcoal is added to said filtered extract in a quantity comprised between 2 and 3 mg per mL of filtered extract and said predetermined quantity correlated to the nature and type of said cereal-based matrix is zero.

15. A method according to any of the previous claims, wherein said fluorophore is 4'-amino-methyl-fluorescein.

16. A method according to claim 15, wherein, prior to the conjugation of DON with 4'-amino-methyl-fluorescein, DON hydroxy groups at C7 and C 15 are protected by reacting DON with 1-butyl-boronic acid.

17. A method according to any of the previous claims, wherein said antibody is the monoclonal antibody having the clone reference number #22, purified as described in Maragos, C. M., and McCormick, S. P., 2000, Monoclonal antibodies for the mycotoxins deoxynivalenol and 3-acetyl-deoxynivalenol. *Food and Agricultural Immunology,* 12, 181-192.

18. A method according to any of the previous claims, wherein said filtered extract is obtained by extraction of said matrix with a PBS buffer and subsequent filtration, the latter being repeated at least once.

19. A method according to any of the previous claims, wherein said filtered extract has undergone a first filtration on a paper filter and a second filtration on a glass microfiber filter.

20. An apparatus (A) for carrying out the method according to any of the previous claims comprising:
- at least a syringe (1) comprising a side union, an active wash flow pump and a needle (2) with a predetermined length;
- a powered unit (3) for moving said syringe by means of a rotary shaft and a mechanical arm (3a);
- at least a drain well (4) to wash said needle (2);
- a first plurality of housings (5) for respective test tubes and/or vials intended to respectively contain the buffer, the antibody and the tracer in solution;
- a second plurality of housings (6) for respective test tubes and/or vials intended to contain each one a respective solution of a filtered extract obtained from one or more cereal-based matrices;
- a fluorescence polarization reader (7) provided with at least a reading cell equipped with a closing lid and with at least a reading cuvette suitable to be housed in said reading cell;
a motor adapted for moving said covering lid;
a programmable computer-controlled management software (8).

21. An apparatus according to claim 18, further comprising grasping means associated to said mechanical arm for automatically removing and replacing said reading cuvette.

22. An apparatus according to claim 21, wherein said grasping means are suitable to directly grasp said reading cuvette or suitable to grasp a stopper which can be associated to said reading cuvette, said stopper being provided with a hole to let said needle (2) pass.

23. An apparatus according to any of claims 18 to 22, further comprising an automatic filtration system to obtain a filtered extract from a solution containing a predetermined quantity of cereal-based matrix..
